# EUROPÄISCHE PATENTSCHRIFT

(11) **EP 2 430 173 B1**
(45) Veröffentlichungstag und Bekanntmachung des Hinweises auf die Patenterteilung: **12.08.2015**
(21) Anmeldenummer: 10720575.9
(22) Anmeldetag: 05.05.2010
(51) Int. Cl.: C12P 7/62, C12P 13/02

(54) **VERFAHREN ZUR HERSTELLUNG VON URETHANGRUPPENHALTIGEN (METH)ACRYLSÄUREESTERN**
METHOD FOR PRODUCING (METH)ACRYLIC ACID ESTERS CONTAINING URETHANE GROUPS
PROCÉDÉ DE PRODUCTION D'ESTERS D'ACIDE (MÉTH)ACRYLIQUE CONTENANT DES GROUPES URÉTHANE

(30) Priorität: 12.05.2009 DE 102009003035; 12.05.2009 US 177291 P
(43) Veröffentlichungstag der Anmeldung: 21.03.2012
(73) Patentinhaber: BASF SE, 67056 Ludwigshafen (DE)
(72) Erfinder: STÜRMER, Rainer, 67127 Rödersheim-Gronau (DE); DREW, Dejana, Houston TX 77006 (US); HAUER, Bernhard, 67136 Fußgönheim (DE); DÄUWEL, Jürgen, 69124 Heidelberg (DE); MEISENBURG, Uwe, 68161 Mannheim (DE)
(86) Internationale Anmeldenummer: PCT/EP2010/056079
(87) Internationale Veröffentlichungsnummer: WO 2010/130608

(56) Entgegenhaltungen:
- WO-A1-2004/050888
- DERANGO R ET AL: "THE LIPASE-CATALYZED SYNTHESIS OF CARBAMOYLOXYETHYL METHACRYLATE", BIOTECHNOLOGY LETTERS, KEW, SURREY, GB, Bd. 16, Nr. 3, 1. März 1994 (1994-03-01), Seiten 241-246, XP009026932, ISSN: 0141-5492, DOI: DOI:10.1007/BF00134619
- PROBST J ET AL: "HOMO- UND COPOLYMERISATION VON N,N-DISUBSTITUIERTEN CARBAMOYLOXYALKYLACRYLATEN UND -METHACRYLATEN", MAKROMOLEKULARE CHEMIE, HUTHIG UND WEPF VERLAG, BASEL, CH, Bd. 177, 1. Januar 1976 (1976-01-01), Seiten 2681-2695, XP009026952, ISSN: 0025-116X, DOI: DOI:10.1002/MACP.1976.021770911

## Beschreibung

Die vorliegende Erfindung betrifft ein Verfahren zur Herstellung von urethangruppenhaltigen (Meth)acrylsäureestern.

Die Herstellung von (Meth)acrylsäureestern erfolgt zumeist durch säure- oder basenkatalysierte Ver- oder Umesterung von (Meth)acrylsäure oder anderen (Meth)acrylsäureestern mit Alkoholen bei Temperaturen von 40 bis deutlich über 100 °C. Aufgrund der hohen Temperaturen ist der Zusatz hoher Mengen von Polymerisationsinhibitoren erforderlich, um eine unerwünschte Polymerisierung der Monomeren zu unterdrücken. Dabei entstehen oft komplexe und bisweilen gefärbte Produktgemische. Zur Entfernung von Färbungen und unumgesetzter Reaktanden werden die Produktgemische durch aufwendige alkalische Wäschen aufgearbeitet. Das Waschverfahren ist langwierig und kostspielig, da sich vor allem teilveresterte Produkte nur langsam extrahieren und abtrennen lassen.

Die Herstellung urethangruppenhaltiger (Meth)acryate über eine konventionelle säurekatalysierte Veresterung ist zudem schwierig, da Urethangruppen säureempfindlich sind.

JP 2001-40039 A beschreibt carbamatgruppenhaltige (Meth)acrylsäureester, die über eine säurekatalysierte Veresterung hergestellt werden. Nachteilig an dem beschriebenen Verfahren ist, dass die Reinheit des erhaltenen Produkts lediglich 75,9 % bei einer Massenbilanz von 95 % beträgt.

EP 136 813 A2 beschreibt die zweistufige Herstellung N-substituierter, carbamatgruppenhaltiger Acrylate durch Umsetzung von mehrfach hydroxyalkylierten Acrylaten mit Isocyanaten. Nachteilig an dem beschriebenen Verfahren ist die Beschränkung auf solche Substrate, die als Isocyanate verfügbar sind. So sind beispielsweise N,N-disubstituierte Carbamate nach diesem Verfahren nicht herstellbar, ebenfalls solche mit N-Substituenten, die gegenüber Isocyanat reaktive Gruppen tragen. Für die Umsetzung mit dem Isocyanat sind zudem toxische Zinnverbindungen als Katalysator notwendig.

Die Herstellung von (Meth)acrylsäureestern durch eine enzymatische Ver- oder Umesterung ist bekannt.

Hajjar et al. beschreiben in Biotechnol. Lett. 1990, 12, 825-830 die enzymatische Umesterung von cyclischen und offenkettigen Alkandiolen mit Ethylacrylat mit einer Lipase aus Chromobacterium viscosum. Die Reaktionen laufen bei einem 18-fachen molaren Überschuss des Alkylacrylats gegenüber dem Diol in einem lösungsmittelfreien System ab. Es entstehen Mischungen aus Mono- und Diacrylaten.

US 5,240,835 beschreibt die Umesterung von Alkylacrylaten mit Alkoholen unter Katalyse eines Biokatalysators aus Corynebacterium oxydans. Beispielhaft wird dort die Reaktion von einem 96-fachen molaren Überschuss Ethylacrylat mit 2,2-Dimethyl-1,3-propandiol aufgeführt. Lediglich 21 % Ausbeute wurden nach 3 Tagen bei 30 °C erhalten.

Derango et al. beschrieben in Biotechnol. Lett. 1994, 16, 241-246 die Lipasekatalysierte Herstellung von Carbamoyloxyethylmethacrylat durch Umesterung von 2-Hydroxyethylcarbamat mit Vinylmethacrylat. Eine vollständige Umsetzung wird erreicht durch das spezielle Edukt Vinylmethacrylat, da freigesetzter Vinylalkohol dem Reaktionsgleichgewicht als Acetaldehyd entzogen wird. Nachteilig an diesem Verfahren ist, dass Vinylmethacrylat nicht kommerziell verfügbar ist.

Aus WO 2004/05088 A1 ist ein weiteres enzymkatalysiertes Herstellungsverfahren urethangruppenhaltiger (Meth)acrylsäureester bekannt. Nachteilig an dem beschriebenen Verfahren ist, dass die Produkte eine relativ geringe Reinheit aufweisen und dennoch ungereinigt weiterverarbeitet werden.

Aufgabe der vorliegenden Erfindung war es daher, ein weiteres, alternatives Verfahren zur Verfügung zu stellen, mit dem urethangruppenhaltige (Meth)acrylsäureester in hohen Umsätzen und hohen Reinheiten aus einfachen, wirtschaftlich zugänglichen Edukten herstellbar sind.

Die Aufgabe wurde gelöst durch ein Verfahren zur Herstellung urethangruppenhaltiger (Meth)acrylsäureester (U) durch Umsetzung eines urethangruppenhaltigen Alkohols (A) mit einem (Meth)acrylsäureester eines gesättigten Alkohols (G) in Gegenwart mindestens eines Polymerisationsinhibitors (P) mit einem Enzym (E) als Katalysator in einem Reaktor, wobei
a) der freiwerdende gesättigte Alkohol und das gegebenenfalls verwendete Schleppmittel mit dem überschüssigen korrespondierenden (Meth)acrylsäureester (G) ein Azeotrop bilden, das Azeotrop unter vermindertem Druck destillativ abgetrennt wird und
b) zumindest ein Teilstrom aus dem Sumpf des Reaktors über den Kopf der Destillationskolonne im Kreis geführt wird.

Mit Hilfe des erfindungsgemäßen Verfahrens ist die Herstellung urethangruppenhaltiger (Meth)acrylsäureester in hoher Reinheit und hohen Ausbeuten und unter milden Bedingungen möglich. Weiterhin tritt keine wesentliche Polymerisatbildung auf. Urethangruppen im Sinne dieser Schrift sind O-substituierte und N-un-, mono- oder disubstituierte Strukturelemente der Formel >N-C(=O)-O-.

(Meth)acrylsäure steht in dieser Schrift für Methacrylsäure und Acrylsäure, bevorzugt für Acrylsäure.

Gesättigt bedeutet im Rahmen dieser Schrift Verbindungen ohne C-C-Mehrfachbindungen (außer selbstverständlich die C=C-Doppelbindung in den (Meth)acryleinheiten).

In dem erfindungsgemäßen Verfahren erfolgt die Umesterung des einem urethangruppenhaltigen Alkohols (A) mit einem (Meth)acrylsäureester eines gesättigten Alkohols (G) in Gegenwart mindestens eines Polymerisationsinhibitors (P) mit einem Enzym (E) als Katalysator, wobei erfindungsgemäß der bei der Umesterung freiwerdende gesättigte Alkohol und das gegebenenfalls verwendete Schleppmittel mit dem überschüssigen korrespondierenden (Meth)acrylsäureester (G) ein Azeotrop bilden, welches über eine dem Reaktor aufgesetzte Kolonne unter vermindertem Druck ausgeschleust und anschließend kondensiert wird. Dabei ist es erfindungswesentlich, dass zumindest ein Teilstrom aus dem Sumpf des Reaktors über den Kopf der Destillationskolonne im Kreis geführt.

Die destillative Abtrennung des Azeotrops über eine dem Reaktor aufgesetzte Kolonne erfolgt erfindungsgemäß unter vermindertem Druck. Der Druck beträgt beispielsweise 20 - 700 mbar, bevorzugt 30 - 500 mbar, besonders bevorzugt 40 - 300 mbar und insbesondere 50 - 150 mbar.

Das abgetrennte Azeotrop wird anschließend kondensiert und vorteilhafterweise direkt in die Anlage zur Herstellung des (Meth)acryläsureesters eines gesättigten Alkohols (G) zugeführt, um es dort in der Veresterung mit (Meth)acrylsäure wiederzuverwerten. Falls zusätzlich ein Schleppmittel verwendet wird, welches ebenfalls als Azeotrop abgetrennt wird, kann dieses Schleppmittel zunächst von dem Azeotrop aus freiwerdendem gesättigten Alkohol und damit korrespondierendem (Meth)acrylsäureester eines gesättigten Alkohols (G) abgetrennt werden, bevor dieses der Veresterung mit (Meth)acrylsäure zugeführt wird.

Weiterhin erfindungswesentlich ist die Kreisführung eines Teilstroms aus dem Sumpf des Reaktors über den Kopf der Destillationskolonne. Bevorzugt beträgt der Austrag des Sumpfstroms, der über den Kopf der Kolonne im Kreis geführt wird, nicht mehr als 50 Gew.-%, bezogen auf das Gesamtgewicht des Sumpfinhalts, bevorzugt nicht mehr als 25 Gew.-%, besonders bevorzugt nicht mehr als 20 Gew.-% und insbesondere nicht mehr als 15 Gew.-%.

Durch die Kreisführung zumindest eines Teilstroms aus dem Sumpf des Reaktors wird vermieden, dass das unstabilisierte Azeotrop in der Kolonne polymerisiert und den Kolonnenkopf verstopft. Auf eine zusätzliche Zugabe von Polymerisationsinhibitor in die Destillationskolonne kann daher vorteilhafterweise in dem erfindungsgemäßen Verfahren verzichtet werden.

Urethangruppenhaltige Alkohole (A) sind solche Verbindungen, die mindestens eine Urethangruppe, bevorzugt 1 bis 10, besonders bevorzugt 1 bis 5, ganz besonders bevorzugt 1 bis 2 und insbesondere eine Urethangruppe, sowie mindestens eine Hydroxygruppe (-OH), bevorzugt 1 bis 10, besonders bevorzugt 1 bis 6, ganz besonders bevorzugt 1 bis 3, insbesondere 1 bis 2 und speziell eine Hydroxygruppe enthalten.

Bevorzugte urethangruppenhaltige Alkohole (A) weisen ein durchschnittliches Molgewicht von 105 bis 800 000 g/mol auf, bevorzugt bis 25 000, besonders bevorzugt 5 000 und ganz besonders bevorzugt bis 4 500 g/mol.

Besonders bevorzugte urethangruppenhaltige Alkohole (A) sind solche, die erhältlich sind durch
a) Umsetzung eines Amins mit einem Carbonat und
b) gegebenenfalls Aufreinigung des aus a) erhältlichen Reaktionsgemisches.

Geeignete Amine für diese Umsetzung sind dabei Ammoniak, primäre oder sekundäre Amine, Carbonate sind O,O'-disubstituierte Carbonate mit dem Strukturemelement -O-C(=O)-O-.

Ganz besonders bevorzugte urethangruppenhaltige Alkohole (A) sind solche, die nach der folgenden Reaktionsgleichung erhältlich sind 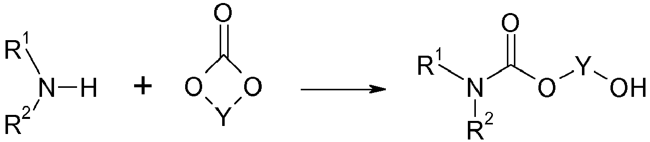 worin
- R¹, R²: unabhängig voneinander Wasserstoff, C₁-C₁₈-Alkyl, gegebenenfalls durch ein oder mehrere Sauerstoff- und/oder Schwefelatome und/oder ein oder mehrere substituierte oder unsubstituierte Iminogruppen unterbrochenes C₂-C₁₈-Alkyl, C₂-C₁₈-Alkenyl, C₆-C₁₂-Aryl, C₅-C₁₂-Cycloalkyl oder einen fünf- bis sechsgliedrigen, Sauerstoff-, Stickstoff- und/oder Schwefelatome aufweisenden Heterocyclus, wobei die genannten Reste jeweils durch Aryl, Alkyl, Aryloxy, Alkyloxy, Heteroatome und/oder Heterocyclen substituiert sein können, oder eine Gruppe der Formel -[Xᵢ]ₖ-H,
- Xᵢ: für jedes i = 1 bis k unabhängig voneinander ausgewählt sein kann aus der Gruppe -CH₂-CH₂-O-, -CH₂-CH₂-N(H)-, -CH₂-CH₂-CH₂-N(H)-, -CH₂-CH(NH₂)-, -CH₂-CH(NHCHO)-, -CH₂-CH(CH₃)-O-, -CH(CH₃)-CH₂-O-, -CH₂-C(CH₃)₂-O-, -C(CH₃)₂-CH₂-O-, -CH₂-CH₂-CH₂-O-, -CH₂-CH₂-CH₂-CH₂-O-, -CH₂-CHVin-O-, -CHVin-CH₂-O-, -CH₂-CHPh-O- und -CHPh-CH₂-O-, worin Ph für Phenyl und Vin für Vinyl steht,
- k: für eine Zahl von 1 bis 50 und
- Y: C₂-C₂₀-Alkylen oder durch ein- oder mehrere Sauerstoff- und/oder Schwefelatome und/oder ein oder mehrere substituierte oder unsubstituierte Iminogruppen und/oder durch eine oder mehrere -(CO)-, -O(CO)O-, -(NH)(CO)O-, -O(CO)(NH)-, -O(CO)- oder -(CO)O-Gruppen unterbrochenes C₂-C₂₀-Alkylen, wobei die genannten Reste jeweils durch Aryl, Alkyl, Aryloxy, Alkyloxy, Heteroatome und/oder Heterocyclen substituiert sein können,
bedeuten.

R¹ und R² können auch gemeinsam einen Ring bilden.

Bevorzugt sind R¹ und R² unabhängig voneinander Wasserstoff, C₁-C₁₂-Alkyl, C₅-C₆-Cycloalkyl oder eine Gruppe der Formel -[Xᵢ]ₖ-H, besonders bevorzugt sind R¹ und R² unabhängig voneinander Wasserstoff, C₁-C₄-Alkyl, C₅-C₆-Cycloalkyl oder eine Gruppe der Formel -[Xᵢ]ₖ-H und ganz besonders bevorzugt Wasserstoff, C₁-C₄-Alkyl, oder eine Gruppe der Formel -[Xᵢ]ₖ-H. Insbesondere ist einer der Reste R¹ und R² Wasserstoff und der andere C₁-C₄-Alkyl, oder eine Gruppe der Formel -[Xᵢ]ₖ-H.

Bevorzugte Xᵢ sind -CH₂-CH₂-O-, -CH₂-CH₂-N(H)-, -CH₂-CH₂-CH₂-N(H)-, -CH₂-CH(NH₂)-, -CH₂-CH(NHCHO)-, -CH₂-CH(CH₃)-O- und -CH(CH₃)-CH₂-O-, besonders bevorzugt ist -CH₂-CH₂-O-, -CH₂-CH₂-N(H)-, -CH₂-CH₂-CH₂-N(H)- und -CH₂-CH(NH₂)-, ganz besonders bevorzugt ist -CH₂-CH₂-O-, -CH₂-CH₂-N(H)- und -CH₂-CH₂-CH₂-N(H)-.

k ist bevorzugt 1 bis 30, besonders bevorzugt 1 bis 20, ganz besonders bevorzugt 1 bis 10 und insbesondere 1 bis 5.

Beispiele für R¹ und/oder R² sind Wasserstoff, Methyl, Ethyl, iso-Propyl, n-Propyl, n-Butyl, iso-Butyl, sek-Butyl, tert-Butyl, n-Hexyl, n-Heptyl, n-Octyl, n-Decyl, n-Dodecyl, n-Tetradecyl, n-Hexadecyl, n-Octadecyl, n-Eicosyl, 2-Ethylhexyl, Cyclopentyl, Cyclohexyl, Cyclooctyl, Cyclododecyl, 2-Hydroxyethyl, 2-Hydroxypropyl, 1-Hydroxypropyl, 5-Hydroxy-3-oxa-pentyl, 8-Hydroxy-3,6-dioxa-octyl oder 11-Hydroxy-3,6,9-trioxa-undecyl.

Y ist bevorzugt C₂-C₁₀-Alkylen, besonders bevorzugt C₂-C₆-Alkylen, ganz besonders bevorzugt C₂-C₄-Alkylen, insbesondere C₂-C₃-Alkylen und speziell C₂-Alkylen, wobei die genannten Reste jeweils durch Aryl, Alkyl, Aryloxy, Alkyloxy, Heteroatome und/oder Heterocyclen substituiert sein können.

Beispiele für Y sind 1,2-Ethylen, 1,2-Propylen, 1,1-Dimethyl-1,2-ethylen, 1-Hydroxymethyl-1,2-ethylen, 2-Hydroxy-1,3-propylen, 1,3-Propylen, 1,4-Butylen, 1,6-Hexylen, 2-Methyl-1,3-Propylen, 2-Ethyl-1,3-Propylen, 2,2-Dimethyl-1,3-Propylen und 2,2-Dimethyl-1,4-butylen, bevorzugt sind 1,2-Ethylen, 1,2-Propylen, 1,3-Propylen, besonders bevorzugt sind 1,2-Ethylen und 1,2-Propylen und ganz besonders bevorzugt ist 1,2-Ethylen.

Beispielhafte Amine sind Ammoniak, Methylamin, Dimethylamin, Ethylamin, Diethylamin, iso-Propylamin, Di-iso-Propylamin, n-Butylamin, Di-n-Butylamin, tert-Butylamin, Monoethanolamin, Diethanolamin, Propanolamin, Dipropanolamin, Piperidin, Piperazin, Pyrrolidin, Cyclopentylamin, Cyclohexylamin, Anilin, Ethylendiamin, Diethylentriamin, Triethylentetramin, Tertraethylenpentamin und Polymere mit Aminfunktion wie in WO 04/050888 A1 auf der Seite 5, ab Zeile 28 bis Seite 6, Zeile 33 beschrieben.

Beispielhafte Carbonate sind Ethylencarbonat, 1,3-Propylencarbonat und 1,2-Propylencarbonat.

Bevorzugte urethangruppenhaltige Alkohole (A) sind solche Verbindungen wie sie in der deutschen Offenlegungsschrift DE 10 2005 016 225 A1 offenbart sind. Von den darin genannten binären Gemischen von strukturisomeren β-Hydroxyalkylcarabamten ist insbesondere das Isomerengemisch von Hydroxypropylcarbamat für das erfindungsgemäße Verfahren bevorzugt. Hydroxypropylcarbamat wird durch Umsetzung von 1,2-Propylencarbonat mit Ammoniak gemäß DE 10 2005 016 255 A1 erhalten.

Die Umsetzung des Amins mit dem Carbonat ist an sich bekannt, beispielsweise aus US 4,820,830 B, Spalte 4, Zeile 44 bis Spalte 5, Zeile 9, und nicht beschränkt.

Typischerweise werden das Amin und das Carbonat in einer Stöchiometrie von 0,7 bis 1,2 mol Amin : 1 mol Carbonat, bevorzugt 0,8 - 1,2 : 1, besonders bevorzugt 0,9 - 1,1:1, ganz besonders bevorzugt 0,95 - 1,1:1 und insbesondere 1:1 mol/mol miteinander umgesetzt. Die Umsetzung erfolgt in der Regel bei einer Temperatur von 0 bis 120 °C, besonders bei 20 bis 100, ganz besonders bevorzugt 30 bis 80 und ganz besonders bevorzugt 40 bis 80 °C. Die Umsetzung ist in der Regel innerhalb von 12 Stunden beendet, bevorzugt innerhalb von 15 Minuten bis 10 Stunden, besonders bevorzugt in 30 Minuten bis 8 Stunden, ganz besonders bevorzugt 45 Minuten bis 6 Stunden und insbesondere innerhalb von 1 bis 4 Stunden.

Die Gesamtaminzahl gemäß DIN 53176 des urethangruppenhaltigen Alkohols (A) sollte nicht mehr als 200 mg KOH/g betragen, vorzugsweise nicht mehr als 100 und ganz besonders bevorzugt nicht mehr als 80 mg KOH/g.

Die Umsetzung des Amins mit dem Carbonat kann ohne Lösungsmittel durchgeführt werden oder in Anwesenheit eines solchen, beispielsweise Alkohole, Ether, Ketone, Kohlenwasserstoffe oder Wasser, bevorzugt ohne Lösungsmittel.

Der urethangruppenhaltige Alkohol (A) kann in einem weiteren Schritt falls gewünscht aufgereinigt werden, beispielsweise durch Filtration, Destillation, Rektifikation, Chromatographie, Behandlung mit Ionentauschern, Adsorbentien, neutraler, saurer und/oder alkalischer Wäsche, Strippen oder Kristallisation.

(Meth)acrylsäureester eines gesättigten Alkohol (G) sind bevorzugt solche Ester von (Meth)acrylsäure mit einem gesättigten C₁-C₁₀-Alkohol.

Beispiele für Verbindungen (G) sind (Meth)acrylsäuremethyl-, -ethyl-, -n-butyl-, -iso-butyl-, n-octyl- und -2-Ethylhexylester, 1,2-Ethylenglycoldi- und -mono(meth)acrylat, 1,4-Butandioldi- und -mono(meth)acrylat, 1,6-Hexandioldi- und -mono(meth)acrylat, Trimethylolpropantri(meth)acrylat und Pentaerythrittetra(meth)acrylat.

Besonders bevorzugt sind (Meth)acrylsäuremethyl-, -ethyl-, -n-butyl- und -2-Ethylhexylester und ganz besonders bevorzugt (Meth)acrylsäuremethyl-, -ethyl- und -n-butylester.

Erfindungsgemäß einsetzbare Enzyme (E) sind beispielsweise ausgewählt unter Hydrolasen, Esterasen (E.C. 3.1.-.-), Lipasen (E.C. 3.1.1.3), Glykosylasen (E.C. 3.2.-.-) und Proteasen (E.C. 3.4.-.-) in freier oder auf einem Träger chemisch oder physikalisch immobilisierter Form, bevorzugt Lipasen, Esterasen oder Proteasen. Besonders bevorzugt sind Novozyme 435 (Lipase aus Candida antartica B) oder Lipase aus Aspergillus sp., Aspergillus niger sp., Mucor sp., Penicilium cyclopium sp., Geotricum candidum sp., Rhizopus javanicus, Burholderia sp., Candida sp., Pseudomonas sp., oder Schweinepankreas, ganz besonders bevorzugt sind Lipase aus Candida antartica B oder aus Burholderia sp.

Der Enzymgehalt im Reaktionsmedium liegt in der Regel im Bereich von etwa 0,1 bis 10 Gew.-%, bezogen auf die Summe der eingesetzten Komponenten (A) und (G). Die Reaktionszeit hängt unter anderem von der Temperatur, der verwendeten Menge und der Aktivität des Enzymkatalysators und vom geforderten Umsatz ab sowie vom urethangruppenhaltigen Alkohol (A). Bevorzugt wird die Reaktionszeit so angepasst, dass der Umsatz aller ursprünglich im Alkohol (A) enthaltenden Hydroxyfunktionen mindestens 70 %, bevorzugt mindestens 80, besonders bevorzugt mindestens 90, ganz besonders bevorzugt mindestens 95 % und insbesondere mindestens 97 % beträgt. In der Regel sind dafür 1 bis 48 Stunden, bevorzugt 1 bis 12 und besonders bevorzugt 1 bis 6 Stunden ausreichend.

Die enzymatische Umesterung mit einem (Meth)acryläsureester eines gesättigten Alkohols (G) erfolgt im Allgemeinen bei 0 bis 100 °C, bevorzugt 20 bis 80 °C, besonders bevorzugt 20 bis 70°C, ganz besonders bevorzugt 20 bis 60 °C.

Das molare Verhältnis von (Meth)acrylsäureester eines gesättigten Alkohols (G) (bezogen auf die (Meth)acryleinheiten) zu urethangruppenhaltigem Alkohol (A) (bezogen auf Hydroxygruppen) kann in einem weiten Bereich, wie z.B. im Verhältnis 100:1 bis 1:1, bevorzugt 50:1 bis 1:1, besonders bevorzugt 20:1 bis 1:1 und ganz besonders bevorzugt 10:1 bis 1:1, schwanken. Bevorzugt liegt der (Meth)acrylsäureester eines gesättigten Alkohols (G) im leichten Überschuss vor, der mit dem freiwerdenden Alkohol als Azeotrop unter vermindertem Druck abdestilliert wird. Auf diese Weise wird das Reaktionsgleichgewicht zugunsten des urethangruppenhaltigen (Meth)acrylsäureesters (U) verschoben.

Gegebenenfalls wird zusätzlich ein Schleppmittel eingesetzt, das mit dem freiwerdenden gesättigten Alkohol und dem damit überschüssigen korrespondierenden (Meth)acrylsäureester (G) ein Azeotrop bildet. Bevorzugt handelt es sich um ein Schleppmittel, dessen mit dem freiwerdenden gesättigten Alkohol und dem damit überschüssigen korrespondierenden (Meth)acrylsäureester (G) gebildetes Azeotrop einen Phasenzerfall zeigt oder welches durch Wasserzugabe gebrochen werden kann. Solche geeigneten Schleppmittel sind beispielsweise n-Pentan, n-Hexan, n-Heptan, Cyclohexan, Methylcyclohexan, Benzol, Toluol, Xylol und beliebige Mischungen davon.

Die Reaktion kann in organischen Lösungsmitteln oder deren Gemischen oder ohne Zusatz von Lösungsmitteln ablaufen. Die Ansätze sind in der Regel weitgehend wasserfrei (d.h. unter 10, bevorzugt unter 5, besonders bevorzugt unter 1 Vol.- % Wasserzusatz).

Der Anteil organischer Lösungsmittel beträgt beispielsweise 0,01-30 Gew.-%, bevorzugt 0,1-5 Gew.-%. Geeignete organische Lösungsmittel sind solche für diese Zwecke bekannten, beispielsweise tertiäre Monoole, wie C₃-C₆-Alkohole, bevorzugt tert-Butanol, tert-Amylalkohol, Pyridin, Poly-C₁-C₄-alkylenglykoldi-C₁-C₄-alkylether, bevorzugt Polyethylenglycoldi-C₁-C₄-alkylether, wie z.B. 1,2-Dimethoxyeethan, Diethylenglycoldimethylether, Polyethylenglycoldimethylether 500, C₁-C₄-Alkylencarbonate, insbesondere Propylencarbonat, C₃-C₆-Alkylessigsäureester, insbesondere tert.-Butylessigsäureester, THF, Toluol, 1,3-Dioxolan, Aceton, iso-Butyl-methylketon, Ethylmethylketon, 1,4-Dioxan, tert-Butylmethylether, Cyclohexan, Methylcyclohexan, Toluol, Hexan, Dimethoxymethan, 1,1-Dimethoxyethan, Acetonitril, sowie deren ein- oder mehrphasige Mischungen.

Wahlweise können zu den organischen Lösungsmitteln wässrige Lösungsmittel zugesetzt werden, so dass - je nach organischem Lösungsmittel - ein- oder mehrphasige Reaktionslösungen entstehen. Beispiele für wässrige Lösungsmittel sind Wasser sowie wässrige, verdünnte (z.B. 10 bis 100 mM) Puffer, beispielsweise mit einem pH-Wert im Bereich von etwa 6 bis 8, wie z.B. Kaliumphosphat- oder TRIS-HCl-Puffer.

Der Wasseranteil im Reaktionsansatz liegt in der Regel bei 0-10 Vol%. Bevorzugt werden die Reaktanden ohne Vorbehandlung (Trocknung, Wasserdotierung) eingesetzt.

Die Substrate liegen entweder gelöst, als Feststoffe suspendiert oder in Emulsion im Reaktionsmedium vor. Vorzugsweise liegt die anfängliche Konzentration der Reaktanden im Bereich von etwa 0,1 bis 20 Mol/l, insbesondere bei 0,15 bis 10 Mol/l oder 0,2 bis 5 Mol/l liegt.

Erfindungsgemäß wird die Reaktion diskontinuierlich durchgeführt. Die Umsetzung kann in allen für eine solche Umsetzung geeigneten Reaktoren durchgeführt werden. Solche Reaktoren sind dem Fachmann bekannt. Bevorzugt erfolgt die Umsetzung in einem Rührkesselreaktor oder einem Festbettreaktor.

Die dem Reaktor aufgesetzte Destillationskolonne ist von an sich bekannter Bauart und weist die üblichen Einbauten auf. Als Kolonneneinbauten kommen prinzipiell alle gängigen Einbauten in Betracht, beispielsweise Böden, Packungen und/oder Schüttungen. Von den Böden sind Glockenböden, Siebböden, Ventilböden, Thormannböden und/oder Dual/Flow-Böden bevorzugt, von den Schüttungen sind solche mit Ringen, Wendeln, Sattelkörpern oder Geflechten bevorzugt. In der Regel sind 5 bis 20 theoretische Böden ausreichend.

Das abdestillierte Azeotrop wird anschließend in einem Kondensator herkömmlicher Bauart kondensiert.

Zur Durchmischung des Reaktionsansatzes können beliebige Verfahren eingesetzt werden. Spezielle Rührvorrichtungen sind nicht erforderlich. Das Reaktionsmedium kann ein- oder mehrphasig sein und die Reaktanden werden darin gelöst, suspendiert oder emulgiert, gegebenenfalls zusammen mit dem Molekularsieb vorgelegt und zum Start der Reaktion, sowie gegebenenfalls ein- oder mehrmals im Verlauf der Reaktion, mit dem Enzympräparat versetzt. Die Temperatur wird während der Reaktion auf den gewünschten Wert eingestellt und kann, falls gewünscht, während des Reaktionsverlauf erhöht oder verringert werden.

Wird die Reaktion im Festbettreaktor durchgeführt, so ist der Festbettreaktor bevorzugt mit immobilisierten Enzymen bestückt, wobei die Reaktionsmischung durch eine mit dem Enzym gefüllte Säule gepumpt wird. Es ist auch möglich, die Umsetzung im Wirbelbett durchzuführen, wobei das Enzym auf einem Träger immobilisiert eingesetzt wird. Die Reaktionsmischung kann kontinuierlich durch die Säule gepumpt werden, wobei mit der Fließgeschwindigkeit die Verweilzeit und damit der gewünschte Umsatz steuerbar ist. Es ist auch möglich, die Reaktionsmischung im Kreislauf durch eine Säule zu pumpen, wobei auch unter Vakuum der freigesetzte Alkohol beziehungsweise das Azeotrop aus freiwerdendem Alkohol mit dem überschüssigen korrespondierenden (Meth)acrylsäureester (G) und mit dem gegebenenfalls verwendeten Schleppmittel gleichzeitig abdestilliert werden kann.

Nach Beendigung der Reaktion kann man das erhältliche Reaktionsgemisch ohne weitere Aufreinigung weiterverwenden oder es erforderlichenfalls in einem weiteren Schritt aufreinigen.

Falls das eingesetzte Enzym (E) nicht in einem Festbettreaktor oder Wirbelbettreaktor immobilisiert vorliegt, wird in der Regel lediglich das eingesetzte Enzym vom Reaktionsgemisch abgetrennt, und das Reaktionsprodukt vom gegebenenfalls verwendeten organischen Lösungsmittel abgetrennt.

Eine Abtrennung vom Enzym erfolgt in einem solchen Fall beispielsweise durch Filtration, Absorption, Zentrifugation oder Dekantieren. Das abgetrennte Enzym kann anschließend für weitere Reaktionen eingesetzt werden.

Die Abtrennung vom organischen Lösungsmittel erfolgt in der Regel durch Destillation, Rektifikation oder bei festen Reaktionsprodukten durch Filtration.

Zur weiteren Aufreinigung des Reaktionsproduktes kann auch eine Chromatographie oder eine Reindestillation durchgeführt werden.

Falls eine Reindestillation zur Aufreinigung des Reaktionsproduktes durchgeführt wird, wird aus dem unter der gegebenenfalls erfolgten Lösungsmitteldestillation anfallenden Sumpf der urethangruppenhaltige (Meth)acrylsäureester (U) in einem weiteren Destillationsschritt als Kopfprodukt isoliert und mit mindestens einem der unten genannten Polymerisationsinhibitoren stabilisiert. Von den dort genannten Stabilisatoren sind für die Reindestillation insbesondere Hydrochinonmonomethylether und Phenothiazin geeignet.

Die für diesen Destillationsschritt verwendbare Rektifikationskolonne ist von bekannter Bauart, wie beispielsweise Füllkörperkolonnen, Packungskolonnen oder Bodenkolonnen, und hat trennwirksame Einbauten (z.B. Glocken-, Sieb- oder Dual-Flow-Böden) oder enthält Schüttungen oder gerichtete Packungen. Diese üblichen Einbauten weisen bevorzugt 10 bis 20 theoretische Böden auf. Auch Dünnschichtverdampfer kommen in Frage. Verdampfer und Kondensator sind ebenfalls herkömmlicher Bauart.

Bevorzugt wird der urethangruppenhaltige (Meth)acrylsäureester (U) bei einer Sumpftemperatur von 100 - 140°C, bevorzugt von 110 - 130°C und einem Kopfdruck von 1 bis 100 mbar, bevorzugt von 1 bis 50 mbar, besonders bevorzugt von 1 bis 10 mbar und insbesondere von 1 bis 5 mbar erhalten.

Zur Stabilisierung kann in den Kondensator eine Lösung von 0,05 - 0,5% Hydrochinonmonomethylether oder ein anderer ähnlich wirksamer Lagerstabilisator eingesprüht werden, wobei die Menge so gewählt wird, dass das Kondensat eine Lagerstabilisatorkonzentration von 10 - 20 ppm aufweist. Ein Teil des Kondensats, bevorzugt 10 - 20%, kann der Kolonne wieder als Rücklauf zugeführt werden.

Der anfallende urethangruppenhaltige (Meth)acrylsäureester (U) hat entsprechend der gaschromatographischen Analyse eine Reinheit von mindestens 98,5%, bevorzugt mindestens 99,0% und besonders bevorzugt mindestens 99,5%.

Das Sumpfprodukt der Reindestillation, das hauptsächlich aus restlichem urethangruppenhaltigen (Meth)acrylsäureester (U), Michael-Additionsprodukten, Stabilisator und Polymeren besteht, kann in eine Rückstandsdestillation und/oder Rückstandsspaltung geleitet werden.

Selbstverständlich ist es auch möglich, die Destillationseinheiten der gegebenenfalls erfolgten Lösungsmitteldestillation und die Reindestillation zu vereinigen. In diesem Fall wird der reine urethangruppenhaltige (Meth)acrylsäureester (U) über einen Seitenabzug, bevorzugt gasförmig, im unteren Kolonnenbereich, bevorzugt in der unteren Hälfte, besonders bevorzugt im unteren Drittel, ausgeschleust, kondensiert und wie oben beschrieben stabilisiert.

Bevorzugt werden im Aufreinigungsschritt jedoch lediglich das eingesetzte Enzym und das gegebenenfalls eingesetzte Lösungsmittel abgetrennt.

Die Reaktionsbedingungen bei der enzymatischen Umesterung sind mild. Aufgrund der niedrigen Temperaturen und sonstigen milden Bedingungen wird die Bildung von Nebenprodukten bei der Umesterung vermieden, die andernfalls zum Beispiel von chemischen Katalysatoren stammen können oder durch unerwünschte radikalische Polymerisation des eingesetzten (Meth)acrylatsäureesters (G), die sonst nur durch Zugabe von Stabilisatoren verhindert werden kann.

Da es sich bei dem in dem erfindungsgemäßen Verfahren eingesetzten (Meth)acrylsäureester eines gesättigten Alkohols (G) als auch bei dem urthangruppenhaltigen (Meth)acrylsäureester (U) um polymerisationsfähige Verbindungen handelt, ist in allen Verfahrensschritten auf eine ausreichende Polymerisationsinhibierung zu achten. Daher findet die Umesterung erfindungsgemäß in Gegenwart mindestens eines Polymerisationsinhibitors (P) statt. Dabei kann es sich um den ohnehin im (Meth)acrylsäureester (G) enthaltenen Lagerstabilisator handeln, es kann aber auch nich ein weiterer Polymerisationsinhibitor zugesetzt werden.

In der Regel werden, bezogen auf die ungesättigten Monomere, je Einzelsubstanz von 1 bis 10 000 ppm, bevorzugt von 10 bis 5 000 ppm, besonders bevorzugt von 30 bis 2 500 ppm und insbesondere von 50 bis 1 500 ppm eines geeigneten Polymerisationsinhibitors (P) eingesetzt.

Geeignete Polymerisationsinhibitoren (P) können beispielsweise N-Oxide (Nitroxyl- oder N-Oxyl-Radikale, also Verbindungen, die wenigstens eine >N-O-Gruppe aufweisen), wie z. B. 4-Hydroxy-2,2,6,6-tetramethylpiperidin-N-oxyl, 4-Oxo-2,2,6,6-tetramethylpiperidin-N-oxyl, 4-Acetoxy-2,2,6,6-tetramethyl-piperidin-N-oxyl, 2,2,6,6-Tetramethylpiperidin-N-oxyl, 4,4',4"-Tris(2,2,6,6-tetramethyl-piperidin-N-oxyl)-phosphit oder 3-Oxo-2,2,5,5-tetramethyl-pyrrolidin-N-oxyl; ein- oder mehrwertige Phenole, die ggf. eine oder mehrere Alkylgruppen aufweisen, wie z. B. Alkylphenole, beispielsweise o-, m- oder p-Kresol (Methylphenol), 2-tert.-Butylphenol, 4-tert.-Butylphenol, 2,4-Di-tert.-butylphenol, 2-Methyl-4-tert.-butylphenol, 2-tert.-Butyl-4-methylphenol, 2,6-tert.-Butyl-4-methylphenol, 4-tert.-Butyl-2,6-dimethylphenol oder 6-tert.-Butyl-2,4-dimethylphenol; Chinone, wie z. B. Hydrochinon, Hydrochinonmonomethylether, 2-Methylhydrochinon oder 2,5-Di-tert.-Butylhydrochinon; Hydroxyphenole, wie beispielsweise Brenzcatechin (1,2-Dihydroxybenzol) oder Benzochinon; Aminophenole, wie z. B. p-Aminophenol; Nitrosophenole, wie z. B. p-Nitrosophenol; Alkoxyphenole, wie beispielsweise 2-Methoxyphenol (Guajacol, Brenzcatechinmonomethylether), 2-Ethoxyphenol, 2-Isopropoxyphenol, 4-Methoxyphenol (Hydrochinonmonomethylether), Mono- oder Di-tert.-Butyl-4-methoxyphenol; Tocipherole, wie z. B. α-Tocopherol sowie 2,3-Dihydro-2,2-dimethyl-7-hydroxybenzofuran (2,2-Dimethyl-7-hydroxycumaran), a-romatische Amine, wie z. B. N,N-Diphenylamin oder N-Nitroso-diphenylamin; Phenylendiamine, wie z. B. N,N'-Dialkyl-p-phenylendiamin, wobei die Alkylreste gleich oder verschieden sein können und jeweils unabhängig voneinander aus 1 bis 4 Kohlenstoffatomen bestehen und geradkettig oder verzweigt sein können, wie z. B. N,N'-Dimethyl-p-phenylendiamin oder N,N'-Diethyl-p-phenylendiamin, Hydroxylamine, wie z.B. N,N-Diethylhydroxylamin, Imine, wie z. B. Methylethylimin oder Methylen violett, Sulfonamide, wie z. B. N-Methyl-4-toluolsulfonamid oder N-tert.-Butyl-4-toluolsulfonamid, Oxime, wie Aldoxime, Ketoxime oder Amidoxime, wie z. B. Diethylketoxim, Methylethylketoxim oder Salicyladoxim, phosphorhaltige Verbindungen, wie z. B. Triphenylphosphin, Triphenylphosphit, Triethylphosphit, Hypophsophorige Säure oder Alkylester der Phosphorigen Säuren; schwefelhaltige Verbindungen wie z. B. Diphenylsulfid oder Phenothiazin; Metallsalze, wie Kupfer- oder Mangan-, Cer-, Nickel-, Chromsalze, beispielsweise -chloride, -sulfate, -salicylate, -tosylate, -acrylate oder-acetate, wie z. B. Kupferacetat, Kupfer(II)chlorid, Kupfersalicylat, Cer(III)acetat oder Cer(III)ethylhexanoat, oder Gemische davon sein.

Bevorzugt wird als Polymerisationsinhibitor(gemisch) mindestens eine Verbindung aus der Gruppe Hydrochinon, Hydrochinonmonomethylether, Phenothiazin, 4-Hydroxy-2,2,6,6-tetramethylpiperidin-N-oxyl, 4-Oxo-2,2,6,6-tetramethylpiperidin-N-oxyl, 2-tert.-Butylphenol, 4-tert.-Butylphenol, 2,4-Di-tert.-Butylphenol, 2-tert.-Butyl-4-methylphenol, 6-tert.-Butyl-2,4-dimethylphenol, 2,6-Di-tert.-Butyl-4-methylphenol, 2-Methyl-4-tert.-butylphenol, Hypophosphorige Säure, Kupferacetat, Kupfer(II)chlorid, Kupfersalicylat und Cer(III)acetat.

Ganz besonders bevorzugt wird Phenothiazin und/oder Hydrochinonmonomethylether (MEHQ) als Polymerisationsinhibitor (P) verwendet.

Zur weiteren Stützung der Stabilisierung wird bevorzugt ein sauerstoffhaltiges Gas, bevorzugt Luft oder ein Gemisch aus Luft und Stickstoff (Magerluft) anwesend sein.

Durch das erfindungsgemäße Verfahren sind in einer bevorzugten Ausführungsform urethangruppenhaltige (Meth)acrylsäureester (U) der Formel (I) zugänglich, 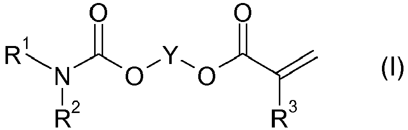 worin
- R¹ und R²: die oben genannten Bedeutungen haben,
- Y: ausgewählt ist unter 1,2-Ethylen, 1,2-Propylen, 1,1-Dimethyl-1,2-ethylen, 1-Hydroxy-methyl-1,2-ethylen, 2-Hydroxy-1,3-propylen, 2-Hydroxy-1,3-propylen, 1,3-Propylen, 1,4-Butylen, 1,6-Hexylen, 2-Methyl-1,3-Propylen, 2-Ethyl-1,3-Propylen, 2,2-Dimethyl-1,3-Propylen und 2,2-Dimethyl-1,4-butylen,
- R³: Wasserstoff oder Methyl, bevorzugt Wasserstoff bedeutet,
mit der Maßgabe, dass mindestens einer der Reste R¹ und R² ungleich Wasserstoff ist.

Ein Vorteil des erfindungsgemäßen Verfahrens ist, dass im wesentlichen vollständige

Umsätze mit einfachen (Meth)acrylsäureestern von gesättigten Alkoholen (G) erzielt werden können, da das Reaktionsgleichgewicht durch die destillative Entfernung des Azeotrops verschoben werden kann.

Die erhältlichen urethangruppenhaltigen (Meth)acrylsäureester (U) können vorteilhaft als Comonomere in Poly(meth)acrylaten oder als Reaktivverdünner in strahlungshärtbaren und/oder Dual-Cure-härtbaren Poly(meth)acrylaten eingesetzt werden. Derartige Poly(meth)acrylate sind als Bindemittel in strahlungs- oder Dual-Cure-härtbaren Beschichtungsmitteln geeignet. So erhältliche Beschichtungen weisen sehr hohe Kratzfestigkeiten, Härten, Chemikalienbeständigkeiten, Elastizität und Haftung, sowohl auf hydrophilen als auch auf hydrophoben Substraten auf.

Eine weitere Verwendung der nach dem erfindungsgemäßen Verfahren hergestellten urethangruppenhaltigen (Meth)acrylsäureester (U) ist als Zusatz in Lackformulierungen möglich. Dabei können die urethangruppenhaltigen (Meth)acrylsäureester (U) sowohl in Basislacken als auch in Decklacken eingesetzt werden. Aufgrund ihrer besonderen Eigenschaften, wie der Erhöhung der Kratzfestigkeit und Elastizität, sowie der Erniedrigung der Viskosität, insbesondere bei verzweigten Polyacrylaten, einer strahlengehärteten Klarlackbeschichtung, ist ihr Einsatz in Deckbeschichtungen bevorzugt.

Für eine solche Verwendung kann der urethangruppenhaltige (Meth)acrylsäureester (U) geeigneterweise mit einem Lösungsmittelzusatz abgemischt werden, um den festen Aggregatzustand zu verhindern und den urethangruppenhaltigen (Meth)acrylsäureester (U) in der flüssigen Phase zu halten. Dazu eignen sich damit mischbare niedere Kohlenwasserstoffe wie Methanol, Ethanol, Propanol, Isopropanol, Butanol, Hexanol und beliebige Mischungen davon. Üblicherweise werden 0 bis 40 Gew.-%, bevorzugt 5 bis 30 Gew.-% und besonders bevorzugt 10 bis 20 Gew.-% eines geeigneten Lösungsmittels, jeweils bezogen auf das Gesamtgewicht von Lösungsmittel und urethangruppenhaltigem (Meth)acrylsäureester (U), eingesetzt.

Die folgenden Beispiele sollen die Eigenschaften der Erfindung erläutern, ohne sie aber einzuschränken.

Falls nicht anders angegeben, bedeuten Prozent immer Gewichtsprozent und Teile immer Gewichtsteile.

### Beispiele

### Beispiel 1

### Herstellung von Hydroxypropylcarbamatacrylat

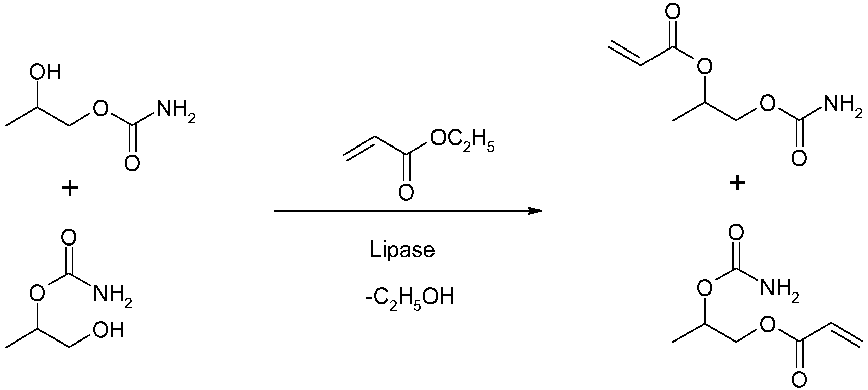

In einem 400L Kessel wurden wurde die Umesterung von Ethylacrylat mit Hydroxypropylcarbamat durchgeführt. Dabei wurden 17,8 kg Hydroxypropylcarbamat (Isomerengemisch) und 145,5 kg Acrylsäureethylester sowie 12 g Hydrochinonmonomethylether vorgelegt. Die gesamte Anlage wurde mit Magerluft (Stickstoff/Sauerstoff-Mischung mit 6 % Sauerstoffanteil) inertisiert. Der im externen Umpumpkreis angeschlossene Enzymreaktor enthielt 1,3 kg Lipase (Novozym^{®} 435). Die Reaktion wurde bei 40 °C und 90 mbar durchgeführt. Das gebildete Ethanol wurde kontinuierlich als Azeotrop mit Acrylsäureethylester über eine Kolonne (Länge I = 150 cm, Durchmesser d = 20 cm, mit Sulzer BX-Packung) abdestilliert. Während der gesamten Reaktionszeit von 24 h wurde ein Strom von 5 kg/h aus dem Kesselsumpf über den Kolonnenkopf gefahren, um Polymerisation zu vermeiden.

Der Umsatz betrug 90 % nach 24 h Reaktionszeit. Anschließend wurde das in Acrylsäureethylester enthaltene Rohprodukt zweimal mit je 1/10 des Gesamtvolumens Wasser gewaschen, wodurch das nicht umgesetzte Edukt entfernt wurde. Man erhielt als Reinprodukt Hydroxypropylcarbamatacrylat in einer Reinheit von > 95 % (GC-Analyse).

### Vergleichsbeispiel 1

In einem 4L Laborreaktor wurde eine analoge Apparatur zu Beispiel 1 im Maßstab 1:100 nachgebildet. Von den Edukten Hydroxypropylcarbamat, Acrylsäureethylester sowie dem Polymerisationsinhibitor Methylhydrochinonmonomethylether und der als Katalysator eingesetzte Lipase (Novozym^{®} 435) wurden jeweils 1/100 der in Beispiel 1 angegeben Mengen eingesetzt. Jedoch wurde auf die Sumpfausschleusung über den Kolonnenkopf verzichtet.

Bereits nach 6 h konnten am Kopf der Kolonnen und an der Packung der Kolonne erste Polymerpartikel in Form von festen Belägen festgestellt werden.

Nach 12 h musste die Reaktion abgebrochen werden, da der obere Teil der Packung durch Polymerisat nahezu undurchlässig geworden war.

## Patentansprüche

1. Verfahren zur Herstellung urethangruppenhaltiger (Meth)acrylsäureester (U) durch Umsetzung eines urethangruppenhaltigen Alkohols (A) mit einem (Meth)acrylsäureester eines gesättigten Alkohols (G) in Gegenwart mindestens eines Polymerisationsinhibitors (P) mit einem Enzym (E) als Katalysator in einem Reaktor, **dadurch gekennzeichnet, dass**
a) der freiwerdende gesättigte Alkohol und das gegebenfalls verwendete Schleppmittel mit dem überschüssigen korrespondierenden (Meth)acrylsäureester (G) ein Azeotrop bilden, das Azeotrop unter vermindertem Druck destillativ abgetrennt wird und
b) zumindest ein Teilstrom aus dem Sumpf des Reaktors über den Kopf der Destillationskolonne im Kreis geführt wird.

2. Verfahren nach Anspruch 1, **dadurch gekennzeichnet, dass** der Druck bei der destillativen Abtrennung des Azeotrops 20 - 700 mbar beträgt.

3. Verfahren nach Anspruch 2, **dadurch gekennzeichnet, dass** der Druck 30 - 500 mbar beträgt.

4. Verfahren nach einem der vorstehenden Ansprüche, **dadurch gekennzeichnet, dass** die enzymatische Umesterung mit einem (Meth)acrylsäureester eines gesättigten Alkohols (G) bei einer Temperatur von 20 - 80°C erfolgt.

5. Verfahren nach einem der vorstehenden Ansprüche, **dadurch gekennzeichnet, dass** das molare Verhältnis von (Meth)acrylsäureester eines gesättigten Alkohols (G) zu urethangruppenhaltigen Alkohol (A) im Bereich von 50:1 bis 1:1 liegt.

6. Verfahren nach einem der vorstehenden Ansprüche, **dadurch gekennzeichnet, dass** der Austrag des Sumpfstroms, der über den Kopf der Kolonne im Kreis geführt wird, nicht mehr als 50 Gew.-%, bezogen auf das Gesamtgewicht des Sumpfinhalts, beträgt.

7. Verfahren nach Anspruch 6, **dadurch gekennzeichnet, dass** der Austrag des Sumpfstroms nicht mehr als 25 Gew.-% beträgt.

8. Verfahren nach einem der vorstehenden Ansprüche, **dadurch gekennzeichnet, dass** der urethangruppenhaltige Alkohol (A) erhältlich ist durch folgende Reaktion 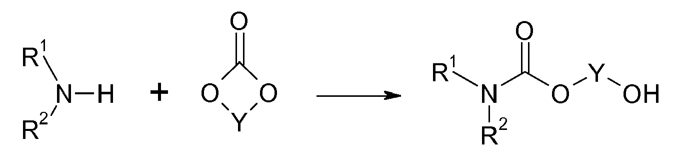 worin
R¹, R² unabhängig voneinander Wasserstoff, C₁-C₁₈-Alkyl, gegebenenfalls durch ein oder mehrere Sauerstoff- und/oder Schwefelatome und/oder ein oder mehrere substituierte oder unsubstituierte Iminogruppen unterbrochenes C₂-C₁₈-Alkyl, C₂-C₁₈-Alkenyl, C₆-C₁₂-Aryl, C₅-C₁₂-Cycloalkyl oder einen fünf- bis sechsgliedrigen, Sauerstoff-, Stickstoff- und/oder Schwefelatome aufweisenden Heterocyclus, wobei die genannten Reste jeweils durch Aryl, Alkyl, Aryloxy, Alkyloxy, Heteroatome und/oder Heterocyclen substituiert sein können, oder eine Gruppe der Formel -[Xᵢ]ₖ-H,
Xi für jedes i = 1 bis k unabhängig voneinander ausgewählt sein kann aus der Gruppe -CH₂-CH₂-O-, -CH₂-CH₂-N(H)-, -CH₂-CH₂-CH₂-N(H)-, -CH₂-CH(NH₂)-, -CH₂-CH(NHCHO)-, -CH₂-CH(CH₃)-O-, -CH(CH₃)-CH₂-O-, -CH₂-C(CH₃)₂-O-, -C(CH₃)₂-CH₂-O-, -CH₂-CH₂-CH₂-O-, -CH₂-CH₂-CH₂-CH₂-O-, -CH₂-CHVin-O-, -CHVin-CH₂-O-, -CH₂-CHPh-O- und -CHPh-CH₂-O-, worin Ph für Phenyl und Vin für Vinyl steht,
k für eine Zahl von 1 bis 50 und
Y C₂-C₂₀-Alkylen oder durch ein- oder mehrere Sauerstoff- und/oder Schwefelatome und/oder ein oder mehrere substituierte oder unsubstituierte Iminogruppen und/oder durch eine oder mehrere -(CO)-, -O(CO)O-, - (NH)(CO)O-, -O(CO)(NH)-, -O(CO)- oder -(CO)O-Gruppen unterbrochenes C₂-C₂₀-Alkylen, wobei die genannten Reste jeweils durch Aryl, Alkyl, Aryloxy, Alkyloxy, Heteroatome und/oder Heterocyclen substituiert sein können,
bedeuten.

9. Verfahren nach einem der vorstehenden Ansprüche, **dadurch gekennzeichnet, dass** der (Meth)acrylsäureester eines gesättigten Alkohols ausgewählt ist aus der Gruppe (Meth)acrylsäuremethyl-, -ethyl-, -n-butyl- und 2-ethylhexylester.

10. Verfahren nach einem der vorstehenden Ansprüche, **dadurch gekennzeichnet, dass** es sich bei dem Enzym (E) um eine Lipase handelt.

## Claims

1. A process for preparing urethane-containing (meth)acrylic esters (U) by reacting a urethane-containing alcohol (A) with a (meth)acrylic ester of a saturated alcohol (G) in the presence of at least one polymerization inhibitor (P) with an enzyme (E) as a catalyst in a reactor, wherein
a) the saturated alcohol released and any entraining agent used form an azeotrope with the excess corresponding (meth)acrylic ester (G), the azeotrope is removed by distillation under reduced pressure and
b) at least a substream from the bottom of the reactor is circulated via the top of the distillation column.

2. The process according to claim 1, wherein the pressure in the distillative removal of the azeotrope is 20-700 mbar.

3. The process according to claim 2, wherein the pressure is 30-500 mbar.

4. The process according to any of the preceding claims, wherein the enzymatic transesterification is effected with a (meth)acrylic ester of a saturated alcohol (G) at a temperature of 20 - 80°C.

5. The process according to any of the preceding claims, wherein the molar ratio of (meth)acrylic ester of a saturated alcohol (G) to urethane-containing alcohol (A) is in the range from 50:1 to 1:1.

6. The process according to any of the preceding claims, wherein the discharge of the bottom stream which is circulated via the top of the column is not more than 50% by weight, based on the total weight of the bottom contents.

7. The process according to claim 6, wherein the discharge of the bottom stream is not more than 25% by weight.

8. The process according to any of the preceding claims, wherein the urethane-containing alcohol (A) is obtainable by the following reaction: 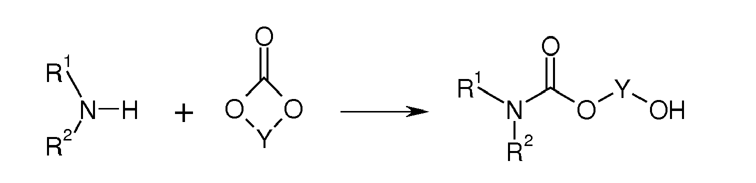 in which
R¹, R² are each independently hydrogen, C₁-C₁₈-alkyl, C₂-C₁₈-alkyl optionally interrupted by one or more oxygen and/or sulfur atoms and/or one or more substituted or unsubstituted imino groups, C₂-C₁₈-alkenyl, C₆-C₁₂-aryl, C₅-C₁₂-cycloalkyl or a five- or six-membered heterocycle having oxygen, nitrogen and/or sulfur atoms, where the radicals mentioned may each be substituted by aryl, alkyl, aryloxy, alkyloxy, heteroatoms and/or heterocycles, or a group of the formula -[Xᵢ]ₖ-H,
Xᵢ for each i = 1 to k may independently be selected from the group of -CH₂-CH₂-O-, -CH₂-CH₂-N(H)-, -CH₂-CH₂-CH₂-N(H)-, -CH₂-CH(NH₂)-, -CH₂-CH(NHCHO)-, -CH₂-CH(CH₃)-O-, -CH(CH₃)-CH₂-O-, -CH₂-C(CH₃)₂-O-, -C(CH₃)₂-CH₂-O-, -CH₂-CH₂-CH₂-O-, -CH₂-CH₂-CH₂-CH₂-O-, -CH₂-CHVin-O-, CHVin-CH₂-O-, -CH₂-CHPh-O- and -CHPh-CH₂-O-, in which Ph is phenyl and Vin is vinyl,
k is from 1 to 50 and
Y is C₂-C₂₀-alkylene or C₂-C₂₀-alkylene interrupted by one or more oxygen and/or sulfur atoms and/or one or more substituted or unsubstituted imino groups and/or by one or more -(CO)-, -O(CO)O-, -(NH)(CO)O-, -O(CO)(NH)-, -O(CO)- or -(CO)O groups, where the radicals mentioned may each be substituted by aryl, alkyl, aryloxy, alkyloxy, heteroatoms and/or heterocycles.

9. The process according to any of the preceding claims, wherein the (meth)acrylic ester of a saturated alcohol is selected from the group of methyl (meth)acrylate, ethyl (meth)acrylate, n-butyl (meth)acrylate and 2-ethylhexyl (meth)acrylate.

10. The process according to any of the preceding claims, wherein the enzyme (E) is a lipase.

## Revendications

1. Procédé pour la préparation d'esters de l'acide (méth)acrylique contenant des groupes uréthane (U) par transformation d'un alcool contenant des groupes uréthane (A) avec un ester de l'acide (méth)acrylique d'un alcool saturé (G) en présence d'au moins un inhibiteur de polymérisation (P) avec une enzyme (E) comme catalyseur dans un réacteur, **caractérisé en ce que**
a) l'alcool saturé qui se libère et l'agent d'entraînement le cas échéant utilisé forment un azéotrope avec l'ester de l'acide (méth)acrylique (G) correspondant en excès, l'azéotrope est éliminé par distillation sous pression réduite et
b) au moins un flux partiel est guidé en circulation du fond du réacteur via la tête de la colonne de distillation.

2. Procédé selon la revendication 1, **caractérisé en ce que** la pression lors de la séparation par distillation de l'azéotrope est de 20-700 mbars.

3. Procédé selon la revendication 2, **caractérisé en ce que** la pression est 30-500 mbars.

4. Procédé selon l'une quelconque des revendications précédentes, **caractérisé en ce que** la transestérification enzymatique avec un ester de l'acide (méth)acrylique d'un alcool saturé (G) a lieu à une température de 20-80°C.

5. Procédé selon l'une quelconque des revendications précédentes, **caractérisé en ce que** le rapport molaire de l'ester de l'acide (méth)acrylique d'un alcool saturé (G) à l'alcool contenant des groupes uréthane (A) se situe dans la plage de 50:1 à 1:1.

6. Procédé selon l'une quelconque des revendications précédentes, **caractérisé en ce que** le produit évacué du flux de fond, qui est guidé en circulation via la tête de la colonne, n'est pas supérieur à 50% en poids par rapport au poids total du contenu du fond.

7. Procédé selon la revendication 6, **caractérisé en ce que** le produit évacué du flux de fond n'est pas supérieur à 25% en poids.

8. Procédé selon l'une quelconque des revendications précédentes, **caractérisé en ce que** l'alcool contenant des groupes uréthane (A) peut être obtenu par la réaction suivante 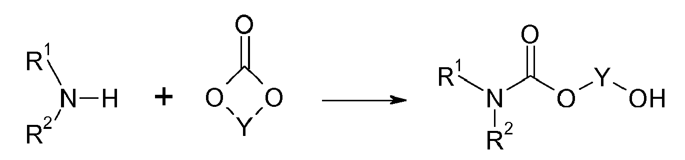 dans laquelle
R¹, R² signifient indépendamment l'un de l'autre hydrogène, alkyle en C₁-C₁₈, alkyle en C₂-C₁₈ éventuellement interrompu par un ou plusieurs atomes d'oxygène et/ou de soufre et/ou un ou plusieurs groupes imino substitués ou non substitués, C₂-C₁₈-alcényle, aryle en C₆-C₁₂, cycloalkyle en C₅-C₁₂ ou hétérocycle de cinq à six chaînons, présentant des atomes d'oxygène, d'azote et/ou de soufre, les radicaux mentionnés pouvant à chaque fois être substitués par aryle, alkyle, aryloxy, alkyloxy, des hétéroatomes et/ou des hétérocycles, ou un groupe de formule -[Xᵢ]ₖ-H,
Xᵢ pour chaque i = 1 à k, peut être choisi, indépendamment l'un de l'autre, dans le groupe formé par -CH₂-CH₂-O-, -CH₂-CH₂-N(H)-, -CH₂-CH₂-CH₂-N(H)-, CH₂-CH(NH₂)-, -CH₂-CH(NHCHO)-, -CH₂-CH(-CH₃)-O-, CH (-CH₃)-CH₂-O-, -CH₂-C(CH₃)₂-O-, -C(CH₃)₂-CH₂-O-, CH₂-CH₂-CH₂-O-, -CH₂-CH₂-CH₂-CH₂-O-, -CH₂-CHVin-O-, CHVin-CH₂-O-, -CH₂-CHPh-O- et -CHPh-OH₂-O-, où Ph représente phényle et Vin représente vinyle,
k vaut un nombre de 1 à 50 et
Y signifie C₂-C₂₀-alkylène ; ou C₂-C₂₀-alkylène interrompu par un ou plusieurs atomes d'oxygène et/ou de soufre et/ou par un ou plusieurs groupes imino substitués ou non substitués et/ou par un ou plusieurs groupes -(CO)-, -O(CO)O-, -(NH) (CO)O-, -O(CO)(NH)-, -O(CO)- ou -(CO)O-, les radicaux mentionnés pouvant être à chaque fois substitués par aryle, alkyle, aryloxy, alkyloxy, des hétéroatomes et/ou des hétérocycles.

9. Procédé selon l'une quelconque des revendications précédentes, **caractérisé en ce que** l'ester de l'acide (méth)acrylique d'un alcool saturé est choisi dans le groupe formé par l'ester méthylique, éthylique, n-butylique et 2-éthylhexylique de l'acide (méth)acrylique.

10. Procédé selon l'une quelconque des revendications précédentes, **caractérisé en ce qu'**il s'agit, pour l'enzyme (E), d'une lipase.
